# EUROPEAN PATENT APPLICATION

(11) **EP 3 117 779 A1**
(43) Date of publication of application: **18.01.2017**
(21) Application number: 15761503.0
(22) Date of filing: 05.02.2015
(51) Int. Cl.: A61B 17/11, A61B 18/12

(54) **TREATMENT DEVICE AND TREATMENT SYSTEM**

(30) Priority: 12.03.2014 JP 2014048912
(71) Applicant: Olympus Corporation, Tokyo 192-8507 (JP)
(72) Inventor: KAWAI, Takanori, Tokyo 192-8507 (JP)
(74) Representative: Gunzelmann, Rainer
(86) International application number: PCT/JP2015/053269
(87) International publication number: WO 2015/137024

(57) **Abstract**

A treatment device includes: a linear member which is deformable between a substantially straight state and a tissue catching state and which is configured to catch a treatment target in a state of being deformed in the tissue catching state; and first and second energy emitting portions provided in the linear member to catch the treatment target in cooperation with each other. The second energy emitting portion is apart from the first energy emitting portion, and is configured to emit energy to the treatment target from between the first energy emitting portion and to treat the treatment target.

## Description

### Technical Field

This invention relates to a treatment device and a treatment system to treat a treatment target of a living tissue by using heat energy.

### Background Art

In various types of surgery, junction techniques to mutually join living tissues are required. For example, a surgical suturing technique and a mechanical anastomotic technique are known as the junction techniques to mutually joint tissues. Moreover, as disclosed in International Publication No. 2011/083027, there has been known a treatment instrument to join tissues of a treatment target by using heat energy (high-frequency energy). The treatment instrument disclosed in International Publication No. 2011/083027 is capable of obtaining a strong joining force earlier when mutually joining tissues in comparison with the case where living tissues are mutually joined by the surgical suturing technique or the mechanical anastomotic technique.

Endoscopic surgery is widely conducted in order to reduce invasive surgical treatment. However, for example, the treatment instrument disclosed in International Publication No. 2011/083027 has high-frequency electrodes facing each other. It is difficult to insert such high-frequency electrodes through a channel of an endoscope. Accordingly, minimally invasive treatment devices capable of obtaining a strong joining force earlier are needed when living tissues are mutually joined.

### Summary of Invention

This invention is intended to provide a treatment device and a treatment system which maintain a minimally invasive state and which can mutually join tissues by a simple procedure.

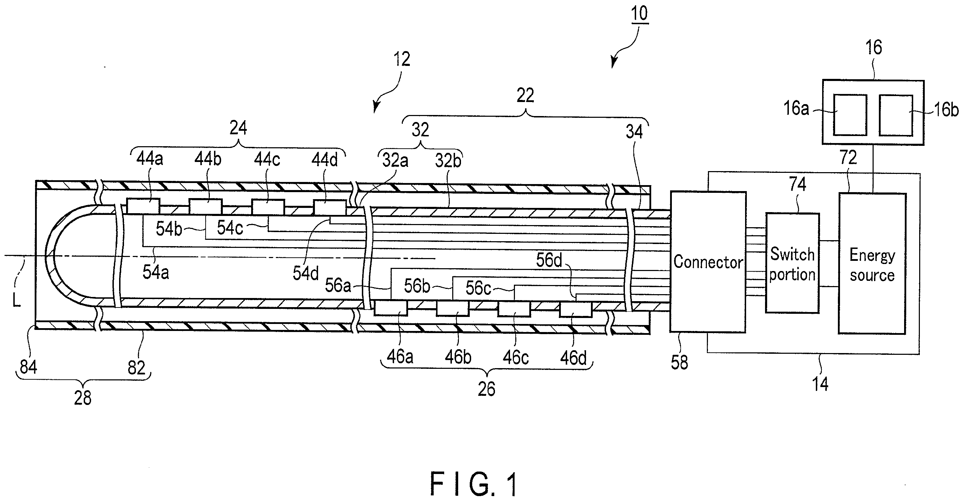

According to one aspect of the present invention, a treatment device includes: a linear member which is deformable between a substantially straight state and a tissue catching state and which is configured to catch a treatment target in a state of being deformed in the tissue catching state; a first energy emitting portion which is provided in the linear member and which is provided at a position to catch the treatment target in a state of the linear member is deformed in the tissue catching state; and a second energy emitting portion which is provided in the linear member in a state of being located apart from the first energy emitting portion, which is provided at a position to catch the treatment target in cooperation with the first energy emitting portion in a state where the linear member is deformed in the tissue catching state, and which is configured to emit energy to the treatment target between the first energy emitting portion and the second energy emitting portion and to treat the treatment target. Brief Description of Drawings
FIG. 1 is a schematic diagram showing a treatment system according to first to third embodiments;

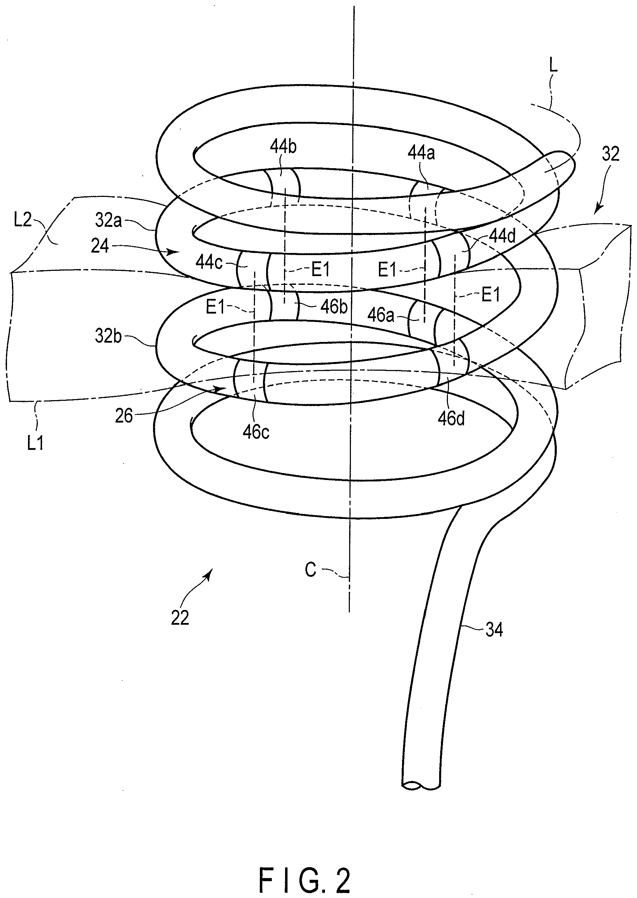

FIG. 2 is a schematic perspective view showing a tissue catching portion (coil portion) of a linear member of a treatment device of the treatment system according to the first embodiment, and showing that energy is emitted between energy emitters which face each other along the central axis of the tissue catching portion;

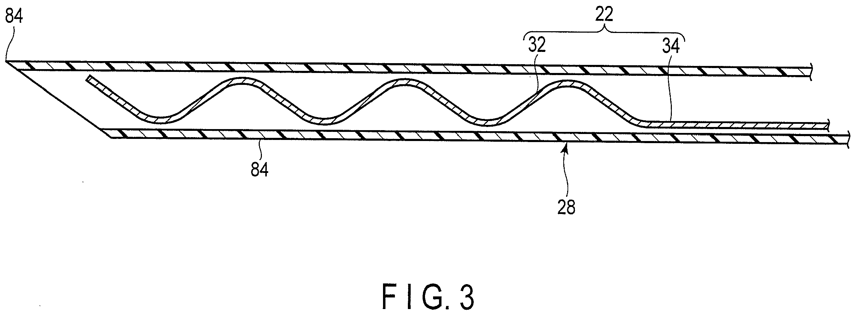

FIG. 3 is a schematic longitudinal sectional view showing that the tissue catching portion and a pusher portion of the linear member of the treatment device of the treatment system according to the first to third embodiments are housed in an applicator;

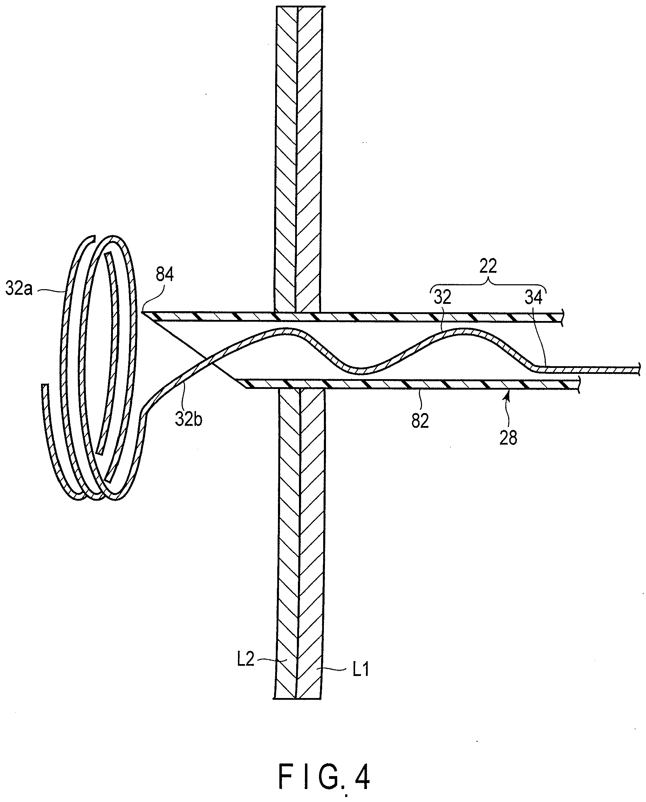

FIG. 4 is a schematic longitudinal sectional view showing that a puncture portion of the applicator of the treatment device of the treatment system according to the first to third embodiments punctures and pierces a living tissue, and then a distal side coil body of the tissue catching portion of the linear member is protruded relative to the distal end of the applicator and then deformed into a coil state (tissue catching state);

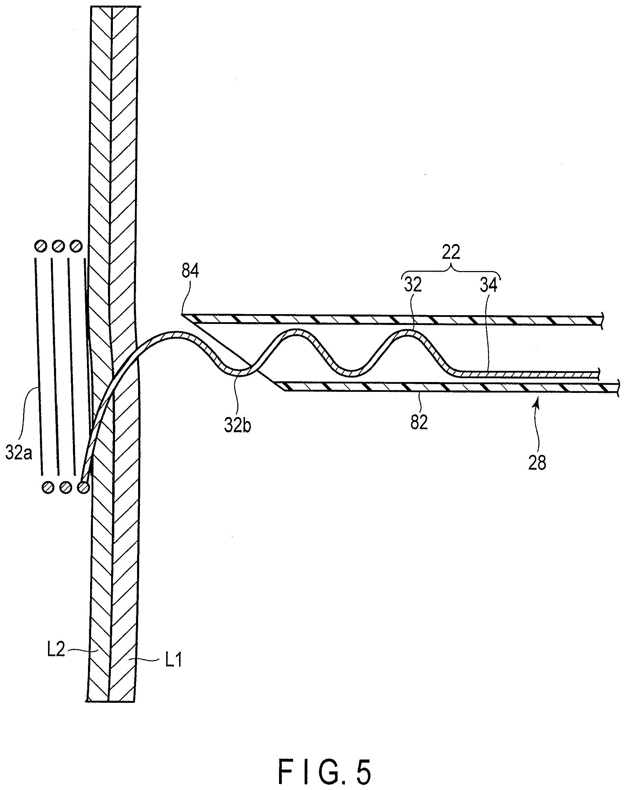

FIG. 5 is a schematic longitudinal sectional view showing that the distal side coil body of the tissue catching portion of the linear member of the treatment device of the treatment system according to the first to third embodiments is deformed into the coil state, and then the puncture portion of the applicator is pulled out;

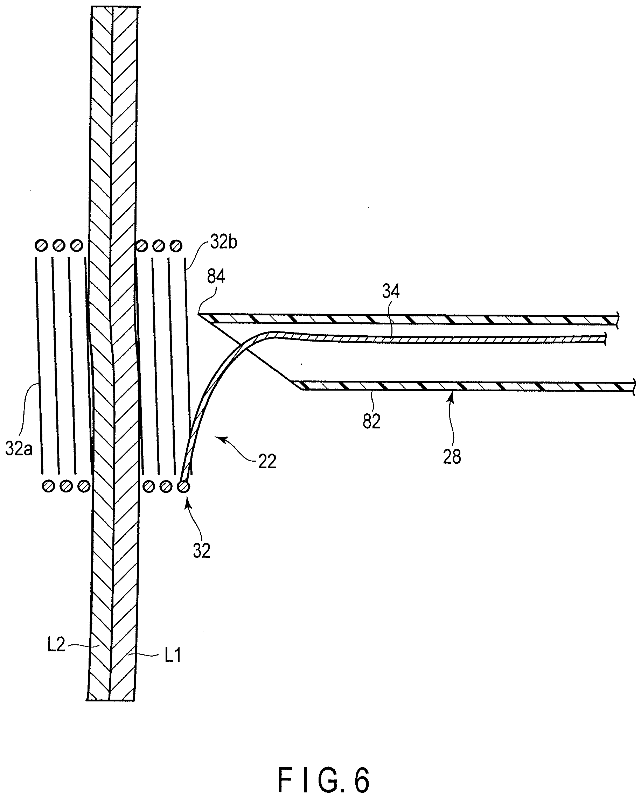

FIG. 6 is a schematic longitudinal sectional view showing that a proximal side coil body of the tissue catching portion of the linear member of the treatment device of the treatment system according to the first to third embodiments is protruded relative to the distal end of the applicator and then deformed into the coil state;

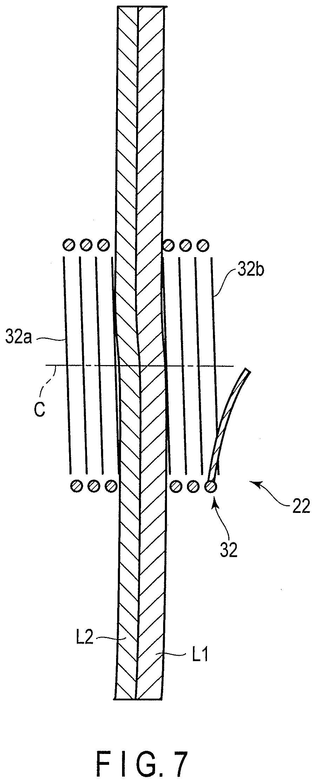

FIG. 7 is a schematic longitudinal sectional view showing that energy is suitably supplied to between the emitters in a state where the tissues are held between a distal side coil and a proximal side coil of the tissue catching portion of the linear member of the treatment device of the treatment system according to the first to third embodiments, to mutually join tissues, and then the linear member is, for example, cut to remove the applicator;

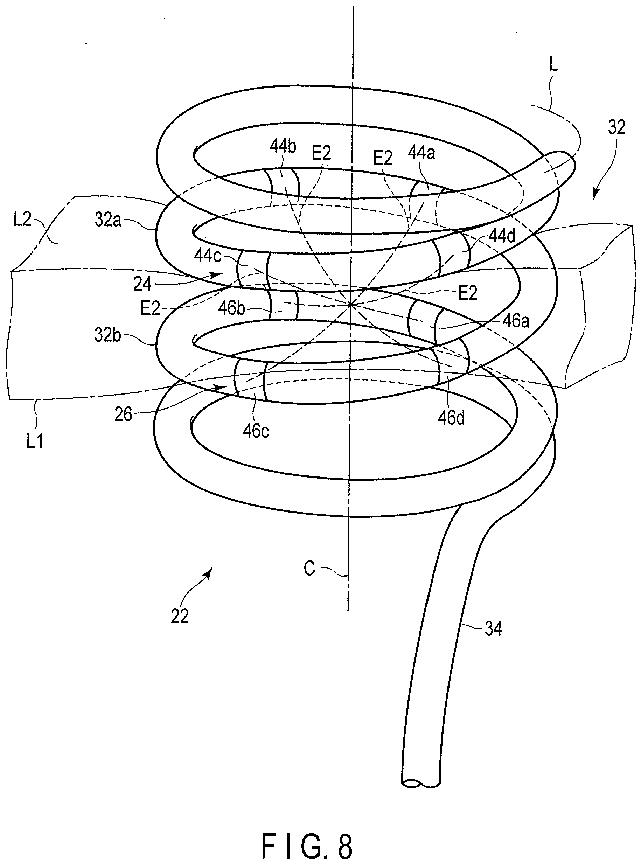

FIG. 8 is a schematic perspective view showing the tissue catching portion of the linear member of the treatment device of the treatment system according to the first embodiment, and showing that energy is emitted to between the energy emitters facing each other across the central axis of the tissue catching portion;

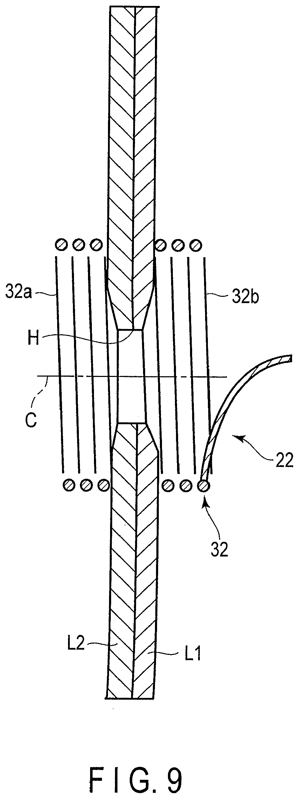

FIG. 9 is a schematic longitudinal sectional view showing that energy is suitably supplied to between the emitters in the state where the tissues are held between the distal side coil and the proximal side coil of the tissue catching portion of the linear member of the treatment device of the treatment system according to the first to third embodiments, to mutually join the tissues and form a hole, and then the linear member is, for example, cut to remove the applicator;

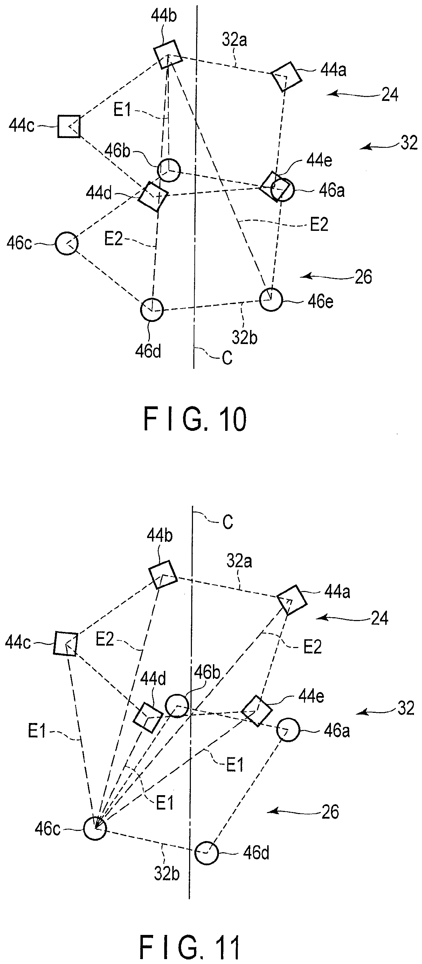

FIG. 10 is a schematic diagram showing the arrangement of first energy emitters and second energy emitters provided in the tissue catching portion of the linear member of the treatment device of the treatment system according to the second embodiment; and
FIG. 11 is a schematic diagram showing the arrangement of first energy emitters and second energy emitters provided in the tissue catching portion of the linear member of the treatment device of the treatment system according to the third embodiment.

### Description of Embodiments

Hereinafter, embodiments of this invention will be described with reference to the drawings.

The first embodiment is described with reference to FIG. 1 to FIG. 9.

It is appropriate that a living tissue treatment system 10 according to this embodiment shown in FIG. 1 be used together with an unshown endoscope. The treatment system 10 includes a living tissue treatment device 12, and a controller (energy source) 14 which controls so that suitable energy may be supplied to later-described first and second energy emitting portions 24 and 26 of the treatment device 12. For example, a foot switch 16 is connected to the controller 14. The foot switch 16 has first and second pedals 16a and 16b. Although described later, a treatment in a joining mode (first mode) to join a first tissue L1 and a second tissue L2 is conducted by way of example when the first pedal 16a is operated. Although described later, a treatment in a hole forming mode (second mode) to form a hole in the first tissue L1 and the second tissue L2 is conducted by way of example when the second pedal 16b is operated. It is also appropriate that a hand switch be used together with or instead of the foot switch 16. It is also appropriate that, for example, push switches equivalent to the first and second pedals 16a and 16b be disposed in a later-described applicator 28.

The treatment device 12 according to this embodiment is formed as a disposable type. The later-described applicator 28 may be reused after being cleaned, disinfected, and sterilized.

The treatment device 12 includes a linear member 22, and the first and second energy emitting portions 24 and 26. The treatment device 12 has the applicator (guide tool) 28 to guide the linear member 22 and the first and second energy emitting portions 24 and 26 to a treatment target. The linear member 22, the first and second energy emitting portions 24 and 26, and the applicator 28 of the treatment device 12 can be inserted through a channel of the unshown endoscope in use, and are appropriately inserted through the channel in use, which will be described later. It is appropriate that the applicator 28 be made of, for example, a resinous material having electric insulating properties.

The linear member 22 has, for example, a coil-shaped tissue catching portion 32, and a pusher portion 34 which can move the catching portion 32 back and forth relative to the distal end (a later-described puncture portion 84) of the applicator 28 along a longitudinal direction. It is appropriate that the holding portion 32 and the pusher portion 34 be integrally formed. The pusher portion 34 may be made of the same material as the holding portion 32, or may be made of a different material.

The catching portion 32 of the wire member 22 is deformable between a substantially straight state and a tissue catching state such as a coil state having spring characteristics, and can catch the treatment target in a state of being deformed in the tissue catching state. When the catching portion 32 is prone to be in the tissue catching state, for example, the coil state, the catching portion 32 has only to exist about several centimeters along a central axis C from its distal end to its proximal end. In a state where the linear member 22 is deformed in the tissue holding state, it is not only difficult to put the linear member 22 into a later-described tubular body 82 of the applicator 28 but also difficult to put the linear member 22 into the channel of the endoscope. When the linear member 22 is put into the tubular body 82 of the applicator 28 and the channel of the endoscope, the linear member 22 is substantially straightened and then put in.

It is appropriate that the catching portion 32 of the linear member 22 be made of a shape-memory alloy such as a nickel-titanium alloy. For example, when heated, the catching portion 32 of the linear member 22 is deformed into the coil-shaped tissue catching state having spring characteristics from the substantially straight state. The linear member 22 made of the shape-memory alloy can be formed by adjusting its composition so that the linear member 22 may maintain the substantially straight state at a temperature (e.g., room temperature) lower than a temperature in a body cavity such as a body temperature and so that the linear member 22 may be deformed into the tissue catching state at the body temperature. A heater may be disposed in the linear member 22 or the later-described puncture portion 84 of the applicator 28 in addition to later-described emitters 44a, 44b, 44c, 44d, 46a, 46b, 46c, and 46d, so that heat may be applied to the linear member 22 from the heater to deform the linear member 22 into the tissue catching state from the substantially straight state. It is also naturally appropriate that the linear member 22 be elastically deformable between the substantially straight state and the tissue catching state (coil state).

In the explanation below, the linear member 22 is made of the shape-memory alloy by way of example.

The tissue catching portion 32 has a distal side coil body (first coil) 32a, and a proximal side coil body *(second coil) 32b provided on the proximal side of the distal side coil body 32a. When the tissue catching portion 32 of the linear member 22 is deformed into the tissue catching state from the substantially straight state, the distal side coil body 32a is located on the distal side along the central axis C of the tissue catching state (coil state), and the proximal side coil body 32b is located adjacent to the proximal side of the distal side coil body 32a. It is appropriate that the distal side coil body 32 and the proximal side coil body 32b be disposed concentrically with the central axis C when the tissue catching portion 32 is in the tissue catching state.

Each of the first and second energy emitting portions 24 and 26 is provided in the tissue catching portion 32 of the linear member 22. Specifically, the first energy emitting portion 24 is provided in the distal side coil body 32a, and the second energy emitting portion 26 is provided in the proximal side coil body 32b. Thus, the first and second energy emitting portions 24 and 26 are located apart from each other.

The first energy emitting portion 24 faces the second energy emitting portion 26 in a state where the tissue catching portion 32 of the linear member 22 is deformed in the tissue catching state. The first energy emitting portion 24 is located at a position to catch the treatment target in cooperation with the second energy emitting portion 26. In other words, the second energy emitting portion 26 is located at a position to catch the treatment target in cooperation with the first energy emitting portion 24 in the state where the catching portion 32 of the linear member 22 is deformed in the tissue catching state. Energy is then emitted to the second energy emitting portion 26 and the first energy emitting portion 24 to treat the treatment target. That is, it is possible to treat the treatment target by emitting energy to the treatment target between the first and second energy emitting portions 24 and 26 through these energy emitting portions.

The first energy emitting portion 24 has multiple (four, in the example described here) energy emitters 44a, 44b, 44c, and 44d (the sign 44 is mainly indicated below). The respective emitters 44 are located apart along a longitudinal direction L of the linear member 22 when the tissue catching portion 32 is in the substantially straight state. In other words, the respective emitters 44 are located apart at suitable intervals along the winding direction L when the distal side coil body 32a of the tissue catching portion 32 is in the tissue catching state (coil state). The respective energy emitters 44 are electrically insulated from each other. For example, a high-frequency electrode or a heater can be used as each energy emitter 44, so that heat energy can be applied to the treatment target. In the example described here, the high-frequency electrodes are used as the energy emitters 44.

The second energy emitting portion 26 has multiple (four, in the example described here) energy emitters 46a, 46b, 46c, and 46d (the sign 46 is mainly indicated below). The respective emitters 46 are located apart along the longitudinal direction L of the linear member 22 when the tissue catching portion 32 is in the substantially straight state. In other words, the respective emitters 46 are located apart at suitable intervals along the winding direction L when the proximal side coil body 32b of the tissue catching portion 32 is in the tissue catching state (coil state). The respective energy emitters 46 are electrically insulated from each other. For example, a high-frequency electrode or a heater can be used as each energy emitter 46, so that heat energy can be applied to the treatment target. In the example described here, the high-frequency electrodes are used as the energy emitters 44.

In this embodiment, the catching portion 32 of the linear member 22_has shape memory. Thus, the energy emitters 44 and 46 which face or substantially face each other in the first and second energy emitting portions 24 and 26 when the linear member 22 is in the tissue catching state are determined. As shown in FIG. 2, when the tissue catching portion 32 is in the tissue catching state, the emitters 44a and 46a face each other, the emitters 44b and 46b face each other, the emitters 44c and 46c face each other, and the emitters 44d and 46d face each other. The emitters 44a and 46a, the emitters 44b and 46b, the emitters 44c and 46c, and the emitters 44d and 46d in particular are disposed to face each other at the positions along the central axis C of the tissue catching state when the linear member 22 is deformed in the tissue catching state. Although described later, the controller 14 controls a later-described switch portion 74 so that energy E1 can be applied across the emitters 44 and 46 that are more proximal among the emitters 44 of the first energy emitting portion 24 and the emitters 46 of the second energy emitting portion 26 when the tissues L1 and L2 are mutually joined.

It is preferable that the emitters 44 and 46 be disposed to face each other across (and also substantially across) the central axis C of the tissue catching state when the linear member 22 is deformed in the tissue catching state. As shown in FIG. 8, the emitters 44a and 46c face each other at the positions across the central axis C, the emitters 44b and 46d face each other at the positions across the central axis C, the emitters 44c and 46a face each other at the positions across the central axis C, and the emitters 44d and 46b face each other at the positions across the central axis C. Although described later, the controller 14 controls the later-described switch portion 74 so that energy E2 can be applied across the emitters 44 and 46 that are more proximal among the emitters 44 of the first energy emitting portion 24 and the emitters 46 of the second energy emitting portion 26 when a hole H is formed in the tissues L1 and L2.

It is appropriate that the linear member 22 be formed into a tubular shape as shown in FIG. 1. Electrical leads 54a, 54b, 54c, and 54d (the sign 54 is mainly indicated below) electrically connected to the first energy emitters 44, and electrical leads 56a, 56b, 56c, and 56d (the sign 56 is mainly indicated below) electrically connected to the second energy emitters 46 are provided inside the linear member 22. That is, the electrical lead 54a is electrically connected to the emitter 44a, the electrical lead 54b is electrically connected to the emitter 44b, the electrical lead 54c is electrically connected to the emitter 44c, the electrical lead 54d is electrically connected to the emitter 44d, the electrical lead 56a is electrically connected to the emitter 46a, the electrical lead 56b is electrically connected to the emitter 46b, the electrical lead 56c is electrically connected to the emitter 46c, and the electrical lead 56d is electrically connected to the emitter 46d. Each of the electrical leads 54 and 56 is attachable to and detachable from the controller 14 having a later-described energy source 72 by a connector 58.

The controller 14 includes the energy source 72 which can supply suitable energy to the first and second energy emitting portions 24 and 26 of the treatment device 12. Thus, the energy output of the energy source 72 is controlled by the controller 14. That is, for example, the energy output amount and energy output time of the energy source 72 are suitably controlled by the controller 14. The energy source 72 is electrically connected to each of the emitters 44 of the first energy emitting portion 24 by each of the electrical leads 54, and also electrically connected to each of the emitters 46 of the second energy emitting portion 26 by each of the electrical leads 56. Thus, energy can be transmitted to each of the emitters 44 and 46 from the energy source 72 through each of the electrical leads 54 and 56.

The energy source 72 in the treatment using high-frequency energy is often limited in electric current capacity. It is thus preferable that the energy source 72 be capable of supplying energy to at least one of the emitters 44 of the first energy emitting portion 24 and at least one of the emitters 46 of the second energy emitting portion 26 in accordance with the electric current capacity. Therefore, as shown in FIG. 1, it is preferable for the controller 14 to have the switch portion 74 which switches the output state in preparation for a possible limit of the electric current capacity. The switch portion 74 is controlled by the controller 14 to select one or more of the emitters 44a, 44b, 44c, and 44d of the first energy emitting portion 24, that is, the electrical leads 54a, 54b, 54c, and 54d, and select one or more of the emitters 46a, 46b, 46c, and 46d of the second energy emitting portion 26, that is, the electrical leads 56a, 56b, 56c, and 56d.

Therefore, energy can be transmitted to suitable energy emitters 44 and 46 among the energy emitters 44 and 46 from the energy source 72 of the controller 14 through the respective electrical leads 54 and 56. It is preferable that energy be simultaneously output to the pairs of emitters 44a, 44b, 44c, 44d, 46a, 46b, 46c, and 46d if the energy source 72 is not limited in electric current capacity.

When the switch portion 74 is controlled by the controller 14, suitable energy is firstly supplied to the emitters 44a and 46a, and then the energy supply is stopped, by way of example. Suitable energy is secondly supplied to the emitters 44b and 46b, and then the energy supply is stopped. Suitable energy is thirdly supplied to the emitters 44c and 46c, and then the energy supply is stopped. Suitable energy is finally supplied to the emitters 44d and 46d, and then the energy supply is stopped. The switch portion 74 is controlled so that energy is substantially continuously supplied to the living tissue. That is, the controller 14 can control so that, for example, the supply of energy to the tissues between the emitters 44b and 46b is started simultaneously or substantially simultaneously with the stopping of the supply of energy to the tissues between the emitters 44a and 46a.

The emitters 44 and 46 can be used as sensors to detect biological information such as impedance between the tissues L1 and L2. Thus, a signal of impedance is input to the controller 14 from the emitters 44 and 46 through the electrical leads 54 and 56. The time of stopping the energy output to the emitters 44 and 46 from the energy source 72 can be automatically controlled by setting a suitable impedance threshold to the controller 14. It is naturally appropriate to set so that energy is output from the energy source 72 only for a suitable time, and the output is stopped when a set time has elapsed. It is also appropriate to set so that the output is stopped when the impedance threshold is not reached after a predetermined time has elapsed since the start of the energy output to the emitters 44 and 46 from the energy source 72.

As shown in FIG. 1 and FIG. 3, the applicator 28 includes the tubular body (sheath) 82 and the puncture portion 84 provided at the distal end of the tubular body 82. The tubular body 82 brings the linear member 22 into the substantially straight state when the linear member 22 is provided in the tubular body 82, and the tubular body 82 houses a protruding portion of the linear member 22 in the tissue catching state when the linear member 22 is protruded from the distal end of the tubular body 82. That is, the applicator 28 has, at its distal end, the puncture portion 84 which can puncture the treatment target. The puncture portion 84 of the applicator 28 can puncture the treatment target and can pierce the treatment target.

Next, functions of the tissue treatment system 10 according to this embodiment are described. In the example described here, energy is emitted to the tissues L1 and L2 from the respective emitters 44 and 46 in order by the use of the switch portion 74.

Firstly described is the case (first mode) to conduct a treatment to join the first tissue L1 and the second tissue L2. Secondly described is the case (second mode) to conduct a treatment to join the first tissue L1 and the second tissue L2 and form the hole H so that the first tissue L1 side and the second tissue L2 side can communicate with each other.

As shown in FIG. 3, the catching portion 32 of the linear member 22 is substantially straightened, and then housed in the tubular body 82 of the applicator 28. As shown in FIG. 1, the connector 58 is connected to the controller 14.

For example, the distal surface of the distal end of an insertion section of the endoscope is put in to face the treatment target in the body cavity. In a state where the distal surface of the distal end of the insertion section of the endoscope is facing the treatment target in the body cavity, the puncture portion 84 of the applicator 28 of the treatment device 12 in which the linear member 22 is in the substantially straight state is placed to face the first tissue L1 of the treatment target through a treatment instrument insertion channel of the endoscope.

As shown in FIG. 4, the first tissue L1 and the second tissue L2 of the treatment target are pierced by the puncture portion 84 of the applicator 28. The distal side coil body 32a of the tissue catching portion 32 of the linear member 22 is then protruded relative to the distal end (puncture portion 84) of the applicator 28 by the pusher portion 34. In this instance, the distal side coil body 32a of the tissue catching portion 32 of the linear member 22 increases in temperature due to, for example, the temperature of the body, and is then deformed into the tissue catching state from the substantially straight state.

As shown in FIG. 5, the puncture portion 84 is pulled out to face the first tissue L1 from the state where the puncture portion 84 is piercing the first tissue L1 and the second tissue L2. Thus, the distal side coil body 32a is left on the second tissue L2 side.

As shown in FIG. 6, the proximal side coil body 32b of the catching portion 32 of the linear member 22 is then protruded relative to the distal end (puncture portion 84) of the applicator 28 by the pusher portion 34. In this instance, the proximal side coil body 32b of the catching portion 32 of the linear member 22 increases in temperature due to, for example, the temperature of the body, and is then deformed into the tissue catching state from the substantially straight state.

Thus, the first tissue L1 and the second tissue L2 are caught between the distal side coil body 32a and the proximal side coil body 32b. That is, the first tissue L1 and the second tissue L2 are caught by the catching portion 32 and thus come into close contact with each other. In this instance, as shown in FIG. 2, the catching portion 32 has shape memory, so that the emitter 44a of the first energy emitting portion 24 and the emitter 46a of the second energy emitting portion 26 face each other, the emitter 44b of the first energy emitting portion 24 and the emitter 46b of the second energy emitting portion 26 face each other, the emitter 44c of the first energy emitting portion 24 and the emitter 46c of the second energy emitting portion 26 face each other, and the emitter 44d of the first energy emitting portion 24 and the emitter 46d of the second energy emitting portion 26 face each other.

In this state, the first pedal 16a of the foot switch 16 connected to the controller 14 is stepped on and operated to conduct, for example, a treatment in the first mode.

As shown in FIG. 2, the controller 14 controls the energy source 72 and the switch portion 74 to output a suitable high-frequency energy (heat energy) across the emitter 44a of the first energy emitting portion 24 and the emitter 46a of the second energy emitting portion 26. Accordingly, the energy E1 is input to the tissues L1 and L2 between the emitters 44a and 46a, and the tissues L1 and L2 are degenerated and thus joined to each other. That is, the controller 14 controls the switch portion 74 to apply the energy E1 across the more proximal emitters 44a and 46a, for example, the emitter 44a and the emitter 46b.

In this instance, biological information such as impedance regarding the tissues L1 and L2 between the emitters 44a and 46a is detected by the emitters 44a and 46a to detect the state of the living tissue. Thus, for example, when the impedance has reached a suitably set threshold, the controller 14 controls the energy source 72 to stop the output of the high-frequency energy.

The controller 14 controls the energy source 72 and the switch portion 74 to output a suitable high-frequency energy across the emitter 44b of the first energy emitting portion 24 and the emitter 46b of the second energy emitting portion 26 so that the tissues L1 and L2 between the emitters 44b and 46b are mutually joined. The high-frequency energy is applied across the emitters 44b and 46b immediately after the end of the application of the high-frequency energy across the emitters 44a and 46a. It is also appropriate that "immediately after" in this instance be simultaneous with the end of the application of the high-frequency energy across the emitters 44a and 46a.

Similarly, the controller 14 applies a suitable output of high-frequency energy across the emitters 44c and 46c immediately after the end of the application of the high-frequency energy across the emitters 44b and 46b. Further, the controller 14 applies a suitable output of high-frequency energy to the emitters 44d and 46d immediately after the end of the application of the high-frequency energy across the emitters 44c and 46c. Thus, the tissues L1 and L2 are mutually joined at the positions between the emitters 44a and 46a, the emitters 44b and 46b, the emitters 44c and 46c, and the emitters 44d and 46d.

That is, the controller 14 controls the energy source 72 and the switch portion 74 to emit the energy E1 to the respective emitters 44 and 46 adjacent to each other in order. Thus, an annular joining portion is formed in the tissues L1 and L2, so that the tissues L1 and L2 are mutually joined.

The controller 14 informs the user that a series of works to join the tissues L1 and L2 has been finished, for example, by sound and/or monitor display. The user releases the first pedal 16a.

The linear member 22 is cut after the output of energy to the emitters 44 and 46 from the energy source 72 has been stopped. In this instance, it is preferable to cut the portion of the linear member 22 protruding from the distal end of the applicator 28. For example, it is preferable to cut the part located in the vicinity of the boundary between the catching portion 32 and the pusher portion 34. This work is performed with, for example, different forceps, which are endoscopically inserted.

The pusher portion 34 of the linear member 22 and the applicator 28 are then pulled out of the channel of the endoscope. Thus, the catching portion 32 of the linear member 22 is left catching the tissues L1 and L2 in a state where the tissues L1 and L2 are mutually joined. The catching portion 32 keeps catching the tissues L1 and L2, and can therefore keep the force of mutually joining the tissues L1 and L2.

In the example described here, the controller 14 controls the switch portion 74 to emit energy to the emitters 44a and 46a, the emitters 44b and 46b, the emitters 44c and 46c, and the emitters 44d and 46d in this order. That is, the controller 14 controls the switch portion 74 to output energy to the emitters 44 and 46 along the winding direction L in the described example. It is also appropriate that energy is emitted to, for example, the emitters 44a and 46a, the emitters 44c and 46c, the emitters 44b and 46b, and the emitters 44d and 46d in this order. That is, the order of energy output to the emitters 44 and 46 may be random if the controller 14 controls the switch portion 74 to simultaneously supply energy to the emitters 44 and 46 that make a pair (that face along the direction of the central axis C), for example, the emitters 44a and 46a.

In FIG. 2, the emitters 44a and 44b, the emitters 44b and 44c, the emitters 44c and 44d, and the emitters 44d and 44a of the first energy emitting portion 24 are illustrated as being located apart from each other by a suitable distance. Similarly, the emitters 46a and 46b, the emitters 46b and 46c, the emitters 46c and 46d, and the emitters 46d and 46a of the second energy emitting portion 26 are illustrated as being located apart from each other by a suitable distance. Thus, for example, when the tissues L1 and L2 are mutually joined, the joining portion can have an annular shape of a broken line rather than an annular shape of a solid line. On the other hand, it is possible to form the annular joining portion of the solid line in the first tissue L1 and the second tissue L2 to mutually join the tissues L1 and L2 by reducing the distance between the emitters 44 and 46 adjacent along the winding direction (the longitudinal direction of the linear member 22) (increasing the areas of the emitters 44 and 46) and suitably controlling the output energy to the emitters 44 and 46.

In the case (second mode) described next, the first tissue L1 and the second tissue L2 are mutually joined and the hole H is formed so that the first tissue L1 side and the second tissue L2 side communicate with each other. The same parts as those with the functions in the first mode are not described.

As shown in FIG. 6, the first tissue L1 and the second tissue L2 are caught between the distal side coil body 32a and the proximal side coil body 32b. That is, the first tissue L1 and the second tissue L2 come into close contact with each other. In this instance, as shown in FIG. 8, the emitter 44a of the first energy emitting portion 24 and the emitter 46a of the second energy emitting portion 26 face each other, the emitter 44b of the first energy emitting portion 24 and the emitter 46b of the second energy emitting portion 26 face each other, the emitter 44c of the first energy emitting portion 24 and the emitter 46c of the second energy emitting portion 26 face each other, and the emitter 44d of the first energy emitting portion 24 and the emitter 46d of the second energy emitting portion 26 face each other.

In this state, the second pedal 16b of the foot switch 16 connected to the controller 14 is stepped on and operated to conduct, for example, a treatment in the second mode.

As shown in FIG. 8, the controller 14 controls the energy source 72 and the switch portion 74 to output a suitable high-frequency energy (heat energy) across the emitter 44a of the first energy emitting portion 24 and the emitter 46c of the second energy emitting portion 26. Accordingly, the energy E2 is input to the tissues L1 and L2 between the emitters 44a and 46c, and the tissues L1 and L2 are degenerated. In this instance, energy density is the highest on the central axis C between the emitters 44a and 46c. That is, the controller 14 controls the switch portion 74 to apply the energy E2 across the more distal emitters 44a and 46c, for example, the emitter 44a and the emitter 46c.

In this instance, biological information such as impedance regarding the tissues L1 and L2 between the emitters 44a and 46c is detected by the emitters 44a and 46c to detect the state of the living tissue. Thus, for example, when the impedance has reached a suitably set threshold, the controller 14 controls the energy source 72 to stop the output of the high-frequency energy. The impedance threshold in the first mode and the impedance threshold in the second mode may be the same or different.

The controller 14 controls the energy source 72 and the switch portion 74 to output a suitable high-frequency energy across the emitter 44b of the first energy emitting portion 24 and the emitter 46d of the second energy emitting portion 26 so that the tissues L1 and L2 between the emitters 44b and 46d are degenerated. The high-frequency energy is applied across the emitters 44b and 46d immediately after the end of the application of the high-frequency energy across the emitters 44a and 46c.

Similarly, the controller 14 applies a suitable output of high-frequency energy to the emitters 44c and 46a immediately after the end of the application of the high-frequency energy to the emitters 44b and 46d. Further, the controller 14 applies a suitable output of high-frequency energy across the emitters 44d and 46b immediately after the end of the application of the high-frequency energy across the emitters 44c and 46a. Thus, the high-frequency energy is applied in a state where energy density on the central axis C of the catching portion 32 is high in the tissues L1 and L2. Therefore, the center (the part on the central axis C) is obliquely cauterized through the energy E2 across the central axis C. Thus, as shown in FIG. 9, the living tissues on the central axis C become necrotic and are removed, and the hole H is formed. The parts around the hole H are annularly and mutually joined by energy density.

That is, the controller 14 controls the energy source 72 and the switch portion 74 to emit the energy E2 to the respective emitters 44 and 46 that are located apart from each other. Thus, in the tissues L1 and L2, the annular hole H is formed on the central axis C, and the parts around the hole H are mutually joined. Therefore, it is possible to form the hole H which keeps the tissues L1 and L2 in communication with each other by using the treatment system 10.

The controller 14 informs the user that a series of works to form the hole H in the tissues L1 and L2 has been finished, for example, by sound and/or monitor display. The user releases the second pedal 16b.

As in the work in the first mode, the linear member 22 is cut after the output of energy to the emitters 44 and 46 from the energy source 72 has been stopped.

The pusher portion 34 of the linear member 22 and the applicator 28 are then pulled out of the channel of the endoscope. Thus, the hole H is formed on the central axis C in the part in which the tissues L1 and L2 are caught by the catching portion 32, and the catching portion 32 of the linear member 22 is left catching the tissues L1 and L2 in a state where the parts around the hole H are joined. The catching portion 32 keeps catching the tissues L1 and L2, and can therefore keep the force of mutually joining the tissues L1 and L2.

In the example described here, the controller 14 controls the switch portion 74 to emit energy to the emitters 44a and 46c, the emitters 44b and 46d, the emitters 44c and 46a, and the emitters 44d and 46b. It is also appropriate that energy is emitted to, for example, the emitters 44a and 46c, the emitters 44c and 46a, the emitters 44b and 46d, and the emitters 44d and 46b in this order. That is, the order of energy output to the emitters 44 and 46 may be random if the controller 14 controls the switch portion 74 to simultaneously supply energy to the emitters 44 and 46 that make a pair (that face each other across the direction of the central axis C), for example, the emitters 44a and 46c.

As described above, the following can be said according to the treatment system 10 in this embodiment.

When surgical suture is endoscopically conducted, work needs to be performed in a narrow space, and therefore a high-level technique is required. The treatment device 12 according to this embodiment can be inserted through the channel of the endoscope in use. This treatment device 12 only requires that after the treatment target is punctured by the applicator 28, the distal side coil body 32a of the tissue catching portion 32 of the linear member 22 be protruded, and the applicator 28 be retracted to protrude the proximal side coil body 32b of the catching portion 32, and then energy be supplied to the emitters 24 and 26 provided in the distal side coil body 32a and the proximal side coil body 32b from the energy source 72 in accordance with a treatment. The work to catch the tissues L1 and L2 can be performed merely by suitably moving the applicator 28 and the linear member 22 in the direction of the central axis C. Therefore, when the treatment device 12 according to this embodiment is used, the work to catch the tissues L1 and L2 can be performed by a simple operation.

It is possible to form the annular joining portion or form the hole H in the treatment target by emitting heat energy from the energy emitting portions 24 and 26 in a state where the treatment target is caught by the tissue catching portion 32 of the linear member 22. Specifically, it is possible to control the energy source 72 and the switch portion 74 by the controller 14 to apply the energy E1 across the more proximal emitters 44a and 46a, for example, the emitter 44a and 46a to form the joining portion. It is also possible to control the energy source 72 and the switch portion 74 by the controller 14 to apply the energy E2 across the more distal emitters 44a and 46c, for example, the emitter 44a and 46c to form the joining portion and form the hole H which allows the first tissue L1 side and the second tissue L2 side to communicate with each other.

Thus, this treatment system 10 conducts a treatment to degenerate the living tissues L1 and L2 by using heat energy, and can therefore considerably reduce the work time when mutually joining the tissues L1 and L2 of the treatment target and when mutually joining the tissues L1 and L2 and also forming the hole H. When mutually joining the tissues and forming the hole H therein, it is not necessary to simply stop blood flow to wait for cells to become necrotic, and it is possible to form the hole H in a short time and mutually join the parts around the hole H by actively inputting heat energy to the living tissue. This hole H can keep the tissue L1 side and the tissue L2 side in communication with each other.

The treatment device 12 according to this embodiment can be endoscopically used, and can therefore maintain a minimally invasive state when treating the treatment target. It is also possible to mutually join tissues to obtain high joining force earlier by a simple procedure. It is also possible to mutually join tissues and form a hole that maintains a communicating state depending on the direction of the input of energy. Similarly, according to the treatment system 10 in this embodiment, it is possible to maintain a minimally invasive state when treating the treatment target, and mutually join tissues to obtain high joining force earlier by a simple procedure. It is also possible to mutually join tissues and form a hole that maintains a communicating state depending on the direction of the input of energy.

In FIG. 2, the emitters 44a and 44b, the emitters 44b and 44c, the emitters 44c and 44d, and the emitters 44d and 44a of the first energy emitting portion 24 are illustrated as being located apart from each other by a suitable distance. Similarly, the emitters 46a and 46b, the emitters 46b and 46c, the emitters 46c and 46d, and the emitters 46d and 46a of the second energy emitting portion 26 are illustrated as being located apart from each other by a suitable distance. It is possible to annularly join the first tissue L1 and the second tissue L2 by reducing the distance between the emitters 44 and 46 adjacent in the winding direction (the longitudinal direction of the linear member 22) L and suitably controlling the output energy to the emitters 44 and 46.

In the example described here, the pairs of emitters 44a, 44b, 44c, 44d, 46a, 46b, 46c, and 46d are switched in order to form the annular joining portion. Depending on the electric current capacity, energy may be simultaneously output from all the emitters 44a, 44b, 44c, 44d, 46a, 46b, 46c, and 46d to form the annular joining portion.

In the example described here, the pairs of emitters 44a, 44b, 44c, 44d, 46a, 46b, 46c, and 46d are switched in order to form the annular joining portion. Depending on the electric current capacity, energy may be simultaneously output from all the emitters 44a, 44b, 44c, 44d, 46a, 46b, 46c, and 46d to form a hole.

Although it has been described that the first mode is one treatment work and the second mode is another treatment work, the joining work in the first mode and the hole making work in the second mode may naturally be a series of treatment works, for example.

The second embodiment is described with reference to FIG. 10. This embodiment is a modification of the first embodiment, and the same components or components having the same functions as those described in the first embodiment are provided with the same reference marks as much as possible and are not described in detail.

In the example described in the first embodiment, the first energy emitting portion 24 has four emitters 44, and the second energy emitting portion 26 has four emitters 46.

As schematically shown in FIG. 10, it is also appropriate that the first energy emitting portion 24 provided in the distal side coil body 32a of the tissue catching portion 32 have five emitters 44a, 44b, 44c, 44d, and 44e, and the second energy emitting portion 26 provided in the proximal side coil body 32b of the tissue catching portion 32 have five emitters 46a, 46b, 46c, 46d, and 46e. That is, the number of the emitters 44 and the number of the emitters 46 may be odd. In this embodiment, for simplification of explanation, the emitters 44 and 46 are located at the vertices of an equilateral pentagon around the central axis (center of gravity) C.

In this case, in the first mode, the controller 14 suitably controls the energy source 72 and the switch portion 74 so that the pair of emitters 44 and 46 facing each other along the direction of the central axis C emit the energy E1 to the tissues between the pair of facing emitters 44 and 46, as has been described in the first embodiment. That is, the controller 14 controls the switch portion 74 to apply the energy E1 across the more proximal emitters 44a and 46a, for example, the emitter 44a and the emitter 46a. The controller 14 then controls the switch portion 74 to emit energy to, for example, the tissues L1 and L2 between the emitters 44b and 46b, between the emitters 44c and 46c, and between the emitters 44d and 46d in order to annularly form a joining portion.

In contrast, in the second mode, for example, the emitter 44b and each of the emitters 46d and 46e make a pair. Thus, in the second mode, the controller 14 suitably controls the energy source 72 and the switch portion 74 so that the energy E2 is emitted to the tissues between the emitters 44b and 46d and the tissues between the emitters 44b and 46e in order. Depending on the electric current capacity, energy may naturally be simultaneously output to the tissues between the emitters 44b and 46d and the tissues between the emitters 44b and 46e. That is, the controller 14 controls the switch portion 74 to apply the energy E2 across the more proximal emitters 44b and 46d and emitters 44b and 46e, for example, the emitters 44b and 46d and the emitters 44b and 46e.

Here, the emitters 44 and 46 in this embodiment do not face each other across the central axis C. Thus, the later-described direction in which the energy E2 flows between the emitters 44 and 46 is skew relative to the central axis C. For example, segments connecting the emitter 44b to the emitters 46d and 46e (parts having the highest energy density in the passages of the energy E2) are skew relative to the central axis C.

When a treatment in the second mode is conducted, the controller 14 switches, by the switch portion 74, the emitters 44 and 46 to emit energy, to the emitter 44a and the emitters 46c and 46d, the emitter 44b and the emitters 46d and 46e, the emitter 44c and the emitters 46e and 46a, the emitter 44d and the emitters 46a and 46b, and the emitter 44e and the emitters 46b and 46c in order. That is, the controller 14 suitably controls the switch portion 74 so that energy can be emitted to a part of the treatment target caught between at least one of the first energy emitters 44 and at least one of the second energy emitters 46. Thus, although the direction in which the energy E2 flows between the emitters 44 and 46 is skew relative to the central axis C, much energy is input to the vicinity of the central axis C, and the energy density in the vicinity of the central axis C is higher. Thus, as in the treatment in the second mode described in the first embodiment, the tissues on the central axis C are cauterized and removed so that the hole H is formed in the tissues L1 and L2. The tissues L1 and L2 around the hole H are mutually joined.

Consequently, as in the first embodiment, the treatment device 12 according to this embodiment can be endoscopically used, and can therefore maintain a minimally invasive state when treating the treatment target, and can also mutually join tissues to obtain high joining force earlier by a simple procedure. It is also possible to mutually join tissues and form a hole that maintains a communicating state depending on the direction of the input of energy. Similarly, according to the treatment system 10 in this embodiment, it is possible to maintain a minimally invasive state when treating the treatment target, and mutually join tissues to obtain a high joining force earlier by a simple procedure. It is also possible to mutually join tissues and form a hole that maintains a communicating state depending on the direction of the input of energy.

The third embodiment is described with reference to FIG. 11. This embodiment is a modification of the first and second embodiments, and the same components or components having the same functions as those described in the first and second embodiments are provided with the same reference marks as much as possible and are not described in detail.

As schematically shown in FIG. 11, it is also appropriate that the first energy emitting portion 24 provided in the distal side coil body 32a of the tissue catching portion 32 have five emitters 44a, 44b, 44c, 44d, and 44e, and the second energy emitting portion 26 provided in the proximal side coil body 32b of the tissue catching portion 32 have four emitters 46a, 46b, 46c, and 46d. That is, the numbers of the emitters 44 and 46 do not need to be the same. The number of the emitters 44 and the number of the emitters 46 may be even or odd.

In this embodiment, for simplification of explanation, each of the emitters 44 of the first energy emitting portion 24 is located at the vertex of an equilateral pentagon around the central axis (center of gravity) C, and each of the emitters 46 of the second energy emitting portion 26 is located at the vertex of a square having the center of gravity on the central axis C.

In this case, in the first mode, for example, one emitter 46c of the second energy emitting portion 26 and two emitters 44c and 44d of the first energy emitting portion 24 make pairs. Thus, the controller 14 suitably controls the energy source 72 and the switch portion 74 so that the energy E1 is emitted to the tissues between the emitters 46c and 44c and the tissues between the emitters 46c and 44d in order. Depending on the electric current capacity, energy may naturally be simultaneously output to the tissues between the emitters 46c and 44c and the tissues between the emitters 46c and 44d.

Here, the emitters 44 and 46 in this embodiment do not face each other parallel to the central axis C, but face each other substantially along the central axis C. Thus, the later-described direction in which the energy E1 flows between the emitters 44 and 46 does not need to be parallel to the central axis C. For example, segments connecting the emitter 44e to the emitters 44c, 44d, and 44e (parts having the highest energy density in the passages of the energy E1) are not parallel to the central axis C. That is, the controller 14 controls the switch portion 74 to apply the energy E1 across the more proximal emitters 44 and 46 out of the emitters 44 of the first energy emitting portion 24 and the emitters 46 of the second energy emitting portion 26.

When a treatment in the first mode is conducted, the controller 14 switches, by the switch portion 74, the emitters 44 and 46 to emit energy, to the emitter 44a and the emitters 44a, 44b, and 44e, the emitter 44b and the emitters 44a, 44b, and 44c, the emitter 44c and the emitters 44c, 44d, and 44e, and the emitter 46d and the emitters 44d and 44e in order by way of example. Thus, although the direction in which the energy E1 flows between the emitters 44 and 46 is not parallel to the central axis C, much energy is input substantially parallel to the central axis C. Thus, as in the treatment in the first mode described in the first embodiment, a joining portion is annularly formed.

In contrast, in the second mode, for example, the emitter 46c and each of the emitters 44a and 44b make a pair. Thus, in the second mode, the controller 14 suitably controls the energy source 72 and the switch portion 74 so that the energy E2 is emitted to the tissues between the emitters 46c and 44a and the tissues between the emitters 46c and 44b in order. Depending on the electric current capacity, energy may naturally be simultaneously output to the tissues between the emitters 46c and 44a and the tissues between the emitters 46c and 44b.

Here, the emitters 44 and 46 in this embodiment do not face each other across the central axis C. That is, the controller 14 controls the switch portion 74 to apply the energy E2 across the more proximal emitters 46c and 44a and emitters 46c and 44b, for example, the emitters 46c and 44a and the emitters 46c and 44b. As has been described in the second embodiment, even if the direction in which the energy E2 flows between the emitters 44 and 46 is skew relative to the central axis C, much energy is input to the vicinity of the central axis C, and the energy density in the vicinity of the central axis C is higher. Thus, as in the treatment in the second mode described in the first embodiment, the tissues on the central axis C are cauterized and removed so that the hole H is formed in the tissues L1 and L2. The tissues L1 and L2 around the hole H are mutually joined.

Consequently, as in the first and second embodiments, the treatment device 12 according to this embodiment can be endoscopically used, and can therefore maintain a minimally invasive state when treating the treatment target, and can also mutually join tissues to obtain high joining force earlier by a simple procedure. It is also possible to mutually join tissues and form a hole that maintains a communicating state depending on the direction of the input of energy. Similarly, according to the treatment system 10 in this embodiment, it is possible to maintain a minimally invasive state when treating the treatment target, and mutually join tissues to obtain a high joining force earlier by a simple procedure. It is also possible to mutually join tissues and form a hole that maintains a communicating state depending on the direction of the input of energy.

Although not shown, the areas of the respective emitters 44 and 46 do not need to be the same.

Although the shape-memory alloy is used for the catching portion 32 of the linear member 22 so that the linear member 22 is deformable between the substantially straight state and the tissue catching state in the example described above in the first embodiment, an elastically deformable component such as a coil spring may be used as the catching portion 32 and brought into the substantially straight state from the tissue catching state and then disposed in the tubular body 82 of the applicator 28. Even when the catching portion 32 is formed as above, the catching portion 32 can catch the tissues L1 and L2 in the same manner as the shape-memory alloy, and energy can be emitted from the energy emitting portions 24 and 26.

### [Additional notes]

The following can be said according to the embodiments described above.

Item 1. A joining method to endoscopically mutually and join living tissues, the method comprising:
using an applicator 28 to pierce treatment target living tissues L1 and L2 from one side (tissue L1 side) to the other side (tissue L2 side);
ejecting a first catching body 32a of a linear member 22 to the other side from the applicator; pulling back the applicator to the one side;
ejecting a second catching body 32b of the linear member 22 to the one side from the applicator, and catching the living tissues L1 and L2 in cooperation with the first catching body;
emitting heat energy to the living tissues between the first and second catching bodies to mutually join the living tissues by the heat energy in a state of catching the living tissues with the first and second catching bodies;
cutting the proximal sides of the first and second catching bodies 32a and 32b in the linear member; and
pulling out the applicator, and pulling out the proximal side of the linear member from the cut position.

Item 2. The method according to Item 1, wherein the heat energy is emitted to between energy emitters close to each other between the first and second catching bodies.

Item 3. The method according to Item 2, wherein emission positions of the heat energy are switched in order, and a joining portion is annularly formed at the end of a treatment.

Item 4. A hole forming method to endoscopically form a hole in living tissues, the method comprising:
using an applicator 28 to pierce treatment target living tissues L1 and L2 from one side (tissue L1 side) to the other side (tissue L2 side);
ejecting a first catching body 32a of a linear member 22 to the other side from the applicator; pulling back the applicator to the one side;
ejecting a second catching body 32b of the linear member 22 to the one side from the applicator, and catching the living tissues L1 and L2 in cooperation with the first catching body;
emitting heat energy to the living tissues between the first and second catching bodies to mutually join the living tissues by the heat energy in a state of catching the living tissues with the first and second catching bodies, and form a hole;
cutting the proximal sides of the first and second catching bodies 32a and 32b in the linear member; and
pulling out the applicator, and pulling out the proximal side of the linear member from the cut position.

Item 5. The method according to Item 1, wherein the heat energy is emitted to between energy emitters located apart from each other between the first and second catching bodies.

Item 6. The method according to Item 5, wherein emission positions of the heat energy are switched in order, and a joining portion is annularly formed and a hole H is formed inside the annular portion at the end of a treatment.

Although the several embodiments have been specifically described above with reference to the drawings, the present invention is not restricted to the foregoing embodiments, and it includes all embodiments carried out without departing from the gist thereof.

### Reference Signs List

10···Living tissue treatment system, 12···Living tissue treatment device, 14···Controller, 22···Linear member, 24··· First energy emitting portion, 26···Second energy emitting portion, 28···Applicator, 32···Tissue catching portion, 32a··· Distal side coil body, 32b···Proximal side coil body, 34··· Pusher portion, 44···First energy emitter, 46···Second energy emitter, 54,56···Electrical leads, 58···Connector, 72···Energy source, 74···**O**utput state switch portion, 82···Tubular body, 84···Puncture portion.

## Claims

1. A treatment device comprising:
a linear member which is deformable between a substantially straight state and a tissue catching state and which is configured to catch a treatment target in a state of being deformed in the tissue catching state;
a first energy emitting portion which is provided in the linear member and which is provided at a position to catch the treatment target in a state of the linear member is deformed in the tissue catching state; and
a second energy emitting portion which is provided in the linear member in a state of being located apart from the first energy emitting portion, which is provided at a position to catch the treatment target in cooperation with the first energy emitting portion in a state where the linear member is deformed in the tissue catching state, and which is configured to emit energy to the treatment target between the first energy emitting portion and the second energy emitting portion and to treat the treatment target.

2. The treatment device according to claim 1, wherein the first and second energy emitting portions are disposed to face each other at positions along a central axis of the tissue catching state or positions substantially along the central axis, in a state where the linear member is deformed in the tissue catching state.

3. The treatment device according to claim 1, wherein the first and second energy emitting portions are disposed to face each other at positions which is across a central axis of the tissue catching state or which is skew relative to the central axis in the state where the linear member is deformed in the tissue catching state.

4. The treatment device according to claim 1, wherein:
the first energy emitting portion includes first energy emitters located apart from each other,
the second energy emitting portion includes second energy emitters located apart from each other, and
energy is emittable to a part of the treatment target caught between at least one of the first energy emitters and at least one of the second energy emitters.

5. The treatment device according to claim 4, wherein energy is emittable to a part caught between one of the first energy emitters and the most proximal energy emitter among the second energy emitters relative to the one of the first energy emitters.

6. The treatment device according to claim 4, wherein energy is emittable to a part caught between one of the first energy emitters and the most distal energy emitter among the second energy emitters relative to the one of the first energy emitters.

7. The treatment device according to claim 4, wherein:
the first energy emitters are electrically insulated from each other, and
the second energy emitters are electrically insulated from each other.

8. The treatment device according to claim 1, further comprising a guide tool which brings the linear member into the substantially straight state when the guide tool is provided inside the linear member and which brings a protruding part of the linear member into the tissue catching state when the linear member is protruded from the distal end of the guide tool.

9. The treatment device according to claim 8, wherein the guide tool includes, at its distal end, a puncture portion configured to puncture the treatment target.

10. The treatment device according to claim 1, wherein the first and second energy emitting portions include high-frequency electrodes.

11. The treatment device according to claim 1, wherein the first and second energy emitting portions include heaters.

12. A treatment system comprising:
the treatment device according to claim 1; and
a controller which includes an energy source configured to supply suitable energy to the first and second energy emitting portions of the treatment device and which controls the energy source.

13. The treatment system according to claim 12, wherein:
the first energy emitting portion of the treatment device includes first energy emitters located apart from each other,
the second energy emitting portion of the treatment device includes second energy emitters located apart from each other, and
the controller is configured to emit energy to at least one selected from the first energy emitters and at least one selected from the second energy emitters.

14. The treatment system according to claim 12, wherein:
the first energy emitting portion of the treatment device includes first energy emitters located apart from each other,
the second energy emitting portion of the treatment device includes second energy emitters located apart from each other, and
the controller includes a switch portion which is switchable to a state where energy flow between the first energy emitters and the second energy emitters along the central axis of the tissue catching state or substantially along the central axis of the tissue catching state when the linear member is in the tissue catching state, and a state where energy flow between the first energy emitters and the second energy emitters which is across the central axis of the tissue catching state or which is skew relative to the central axis of the tissue catching state.
